# EUROPEAN PATENT SPECIFICATION

(11) **EP 1 164 131 B1**
(45) Date of publication and mention of the grant of the patent: **07.09.2005**
(21) Application number: 01114372.4
(22) Date of filing: 13.06.2001
(51) Int. Cl.: C07D 213/80, C07C 45/34, C07C 29/50, C07C 35/36, B01J 31/02, C07D 207/46

(54) **Process for producing organic compounds by catalysis of imide compounds**
Verfahren zur Herstellung organischer Verbindungen unter Katalyse von Imidverbindungen
Procédé pour la préparation de composés organiques par catalyse de composés imides

(30) Priority: 14.06.2000 JP 2000179185
(43) Date of publication of application: 19.12.2001
(73) Proprietor: DAICEL CHEMICAL INDUSTRIES, LTD., Osaka 590-8501 (JP)
(72) Inventor: Kitayama, Kenji, Himeji-shi, Hyogo 671-1262 (JP); Tatsumi, Atsuo, Himeji-shi, Hyogo 671-1134 (JP); Terada, Masahiko, Himeji-shi, Hyogo 671-1213 (JP); Hirai, Naruhisa, Himeji-shi, Hyogo 671-1226 (JP)
(74) Representative: Grünecker, Kinkeldey, Stockmair & Schwanhäusser Anwaltssozietät

(56) References cited:
- EP-A- 0 824 962
- EP-A- 0 878 458
- EP-A- 0 897 747
- EP-A- 0 990 631
- EP-A- 0 990 634
- US-A- 5 030 739
- US-B1- 6 229 050
- PATENT ABSTRACTS OF JAPAN vol. 1996, no. 06, 28 June 1996 (1996-06-28) & JP 08 038909 A (DAICEL CHEM IND LTD ET AL.), 13 February 1996 (1996-02-13)

## Description

### BACKGROUND OF THE INVENTION

### 1. Field of the Invention

The present invention relates to processes for producing organic compounds by catalysis of imide compounds such as N-hydroxyphthalimide.

### 2. Description of the Related Art

N-hydroxyphthalimide and other imide compounds serve as catalysts to smoothly proceed reactions under mild conditions and have received attention. Such reactions catalyzed by imide compounds include, for example, oxidation by molecular oxygen, carboxylation, nitration, sulfonation, and carbon-carbon bond formation reactions (e.g., acylation and radical coupling reactions).

For example, Japanese Unexamined Patent Application Publications No. 8-38909 and No. 9-327626 each disclose a process in which hydrocarbons, alcohols, or other substrates are oxidized by molecular oxygen in the presence of an imide compound catalyst to yield, for example, corresponding alcohols, aldehydes, ketones, or carboxylic acids. Japanese Unexamined Patent Application Publication No. 9-278675 discloses a process for oxidation of conjugated compounds by catalysis of the imide compound catalyst. Japanese Unexamined Patent Application Publication No. 10-316610 mentions that oxidation of ethers in the presence of the imide compound catalyst yields, for example, esters, acid anhydrides, or lactones. PCT International Publication No. WO99/50204 describes a process, in which compounds each having a non-aromatic ethylenic bond are oxidized by molecular oxygen in the presence of the imide compound catalyst and a co-oxidizing (co-oxidizable) agent to yield corresponding epoxides, and a process, in which ketones are oxidized by molecular oxygen in the presence of the imide compound catalyst and a co-oxidizing (co-oxidizable) agent to yield corresponding esters or lactones.

Japanese Unexamined Patent Application Publication No. 11-239730 discloses a process, in which substrates are allowed to react with nitrogen oxides in the presence of the imide compound catalyst to yield corresponding nitro compounds, and a process, in which substrates are allowed to react with carbon monoxide and oxygen in the presence of the aforementioned catalyst to yield corresponding carboxylic acids. PCT International Publication No. WO99/41219 mentions that the reaction of a substrate with oxygen and a 1,2-dicarbonyl compound such as biacetyl in the presence of the imide compound catalyst permits an acylation reaction to proceed under mild conditions. The Chemical Society of Japan, Spring Annual Meeting, Lecture Proceedings (1999) reports that the reaction of an α,β-unsaturated ester with an alcohol and oxygen by catalysis of N-hydroxyphthalimide permits a radical coupling reaction to proceed to thereby yield an α-hydroxy-γ-butyrolactone in a good yield. The Lecture Proceedings also reports that the reaction of a hydrocarbon such as adamantane with oxygen and sulfur dioxide by catalysis of N-hydroxyphthalimide yields a corresponding sulfonic acid.

As is described above, the imide compound catalysts are very useful as catalysts for wide variety of organic synthesis reactions such as oxidation reactions, and demands have been made to provide processes for producing target compounds by catalysis of the imide compound catalysts with higher conversion or selectivity.

### SUMMARY OF THE INVENTION

Accordingly, an object of the present invention is to provide a process for producing an organic compound by catalysis of an imide compound catalyst such as N-hydroxyphthalimide, which process can produce the target compound with higher conversion or selectivity.

After intensive investigations to achieve the above object, the present inventors have found that the addition of the imide compound catalyst to a reaction system in installments to perform a reaction can markedly improve the conversion of a reaction material (substrate) and/or the selectivity of a target compound. The present invention has been accomplished based on these findings.

The present invention provides a process for producing an organic compound by a reaction selected from the group consisting of oxidation reactions, carboxylation reactions, nitration reactions, sulfonation reactions, and carbon-carbon bond formation reactions, by catalysis of an imide compound represented by the following Formula (1): 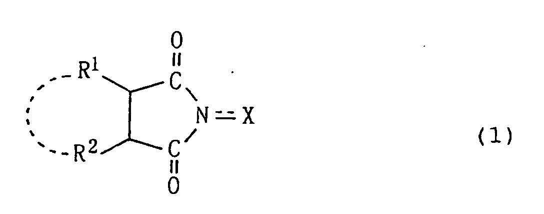 wherein each of R¹ and R² is independently a hydrogen atom, a halogen atom, a straight- or branched-chain alkyl group having from 1 to 10 carbon atoms, a phenyl or naphthyl group, a cyclopentyl, cyclohexyl, or cyclooctyl group, a hydroxyl group, an alkoxy group having from 1 to 10 carbon atoms, a carboxyl group, an alkoxycarbonyl group having from 1 to 10 carbon atoms in the alkoxy moiety, or an acyl group having from 1 to 6 carbon atoms, where R¹ and R² may be combined to form a double bond or an aromatic or non-aromatic ring having from 5 to 12 members; X is an oxygen atom or a hydroxyl group; and one or two of N-substituted cyclic imido group indicated in the formula may be further formed on said R¹, R², or on the double bond or aromatic or non-aromatic ring formed together by R¹ and R², characterised in that the imide compound catalyst is added to a reaction system in installments.

The present invention also relates to the use of an imide compound of Formula (1) as defined above as a catalyst for the production of an organic compound, wherein said imide compound catalyst is added to a reaction system in installments to perform a reaction selected from the group consisting of oxidation reactions, carboxylation reactions, nitration reactions, sulfonation reactions, and carbon-carbon bond formation reactions.

### DESCRIPTION OF THE PREFERRED EMBODIMENTS

### [Imide Compounds]

Of the substituents R¹ and R² in the imide compounds of Formula (1), the halogen atom includes iodine, bromine, chlorine and fluorine. The alkyl group includes methyl, ethyl, propyl, isopropyl, butyl, isobutyl, s-butyl, t-butyl, pentyl, hexyl, heptyl, octyl, decyl, and other straight- or branched-chain alkyl groups each having from 1 to 10 carbon atoms. Preferred alkyl groups are alkyl groups each having from 1 to 6 carbon atoms, of which lower alkyl groups each having from 1 to 4 carbon atoms are typically preferred.

Illustrative alkoxy groups are methoxy, ethoxy, propoxy, isopropoxy, butoxy, isobutoxy, t-butoxy, pentyloxy, hexyloxy, and other alkoxy groups each having from 1 to 10 carbon atoms, and preferably having from 1 to 6 carbon atoms. Among them, lower alkoxy groups each having from 1 to 4 carbon atoms are typically preferred.

Examples of the alkoxycarbonyl group include methoxycarbonyl, ethoxycarbonyl, propoxycarbonyl, isopropoxycarbonyl, butoxycarbonyl, isobutoxycarbonyl, t-butoxycarbonyl, pentyloxycarbonyl, hexyloxycarbonyl, and other alkoxycarbonyl groups each having from 1 to 10 carbon atoms in the alkoxy moiety. Preferred alkoxycarbonyl groups are alkoxycarbonyl groups each having from 1 to 6 carbon atoms in the alkoxy moiety, of which lower alkoxycarbonyl groups each having from 1 to 4 carbon atoms in the alkoxy moiety are typically preferred.

Illustrative acyl groups include formyl, acetyl, propionyl, butyryl, isobutyryl, valeryl, isovaleryl, pivaloyl, and other acyl groups each having from 1 to 6 carbon atoms.

The substituents R¹ and R² may be identical to or different from each other. The substituents R¹ and R² in Formula (1) may be combined to form a double bond, or an aromatic or non-aromatic ring. The aromatic or non-aromatic ring has from 5 to 12 members, and specifically from 6 to 10 members. The ring may be a heterocyclic ring or condensed heterocyclic ring, but it is often a hydrocarbon ring. Such rings include, for example, non-aromatic alicyclic rings (e.g., cyclohexane ring and other cycloalkane rings which may have a substituent, cyclohexene ring and other cycloalkene rings which may have a substituent), non-aromatic bridged rings (e.g., 5-norbornene ring and other bridged hydrocarbon rings which may have a substituent), benzene ring, naphthalene ring, and other aromatic rings (including condensed rings) which may have a substituent. The ring is composed of an aromatic ring in many cases. The ring may have a substituent. Examples of substituents include alkyl groups, haloalkyl groups, hydroxyl group, alkoxy groups, carboxyl group, alkoxycarbonyl groups, acyl groups, nitro group, cyano group, amino group, and halogen atoms.

In Formula (1), X represents an oxygen atom or a hydroxyl group, and the bond between the nitrogen atom N and X is a single bond or a double bond.

One or two of the N-substituted cyclic imido group indicated in Formula (1) may be further formed on R¹, R², or on the double bond or aromatic or non-aromatic ring formed together by R¹ and R². For example, when R¹ or R² is an alkyl group having two or more carbon atoms, the N-substituted cyclic imido group may be formed together with the adjacent two carbon atoms constituting the alkyl group. Likewise, when R¹ and R² are combined to form a double bond, the N-substituted cyclic imido group may be formed together with the double bond. When R¹ and R² are combined to form an aromatic or non-aromatic ring, the N-substituted cyclic imido group may be formed with the adjacent two carbon atoms constituting the ring. Each of these imide compounds of Formula (1) can be used alone or in combination in the reaction.

Preferred imide compounds include compounds of the following formulae: 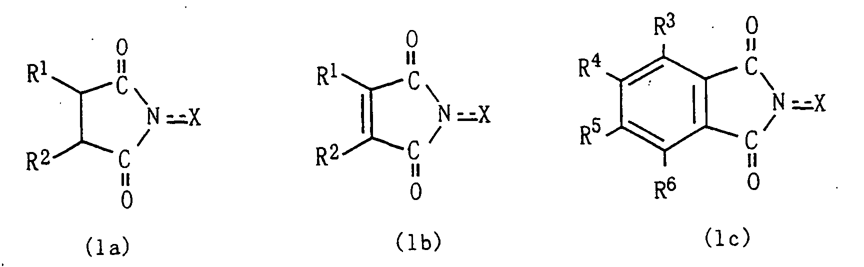 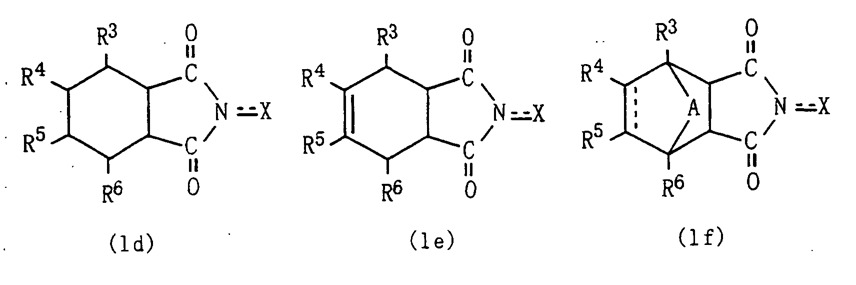 wherein R³ to R⁶ are each independently a hydrogen atom, an alkyl group, a haloalkyl group, a hydroxyl group, an alkoxy group, a carboxyl group, an alkoxycarbonyl group, an acyl group, a nitro group, a cyano group, an amino group, or a halogen atom, where adjacent groups of R³ to R⁶ may be combined to form an aromatic or non-aromatic ring; in Formula (1f), A is a methylene group or an oxygen atom, and R¹, R² and X have the same meanings as defined above, where one or two of N-substituted cyclic imido group indicated in Formula (1c) may be further formed on the benzene ring in Formula (1c).

In the substituents R³ to R⁶, the alkyl group includes similar alkyl groups to those exemplified above, of which alkyl groups each having 1 to 6 carbon atoms are specifically preferred. The haloalkyl group includes trifluoromethyl group and other haloalkyl groups each having from 1 to 4 carbon atoms. The alkoxy group includes similar alkoxy groups to those mentioned above, of which lower alkoxy groups each having from 1 to 4 carbon atoms are specifically preferred. The alkoxycarbonyl group includes similar alkoxycarbonyl groups to those described above, of which lower alkoxycarbonyl groups each having from 1 to 4 carbon atoms in the alkoxy moiety are specifically preferred. The acyl group includes similar acyl groups to those described above, of which acyl groups each having from 1 to 6 carbon atoms are specifically preferred. The illustrative halogen atoms include fluorine, chlorine and bromine atoms. Each of the substituents R³ to R⁶ is often a hydrogen atom, a lower alkyl group having from 1 to 4 carbon atoms, a carboxyl group, a nitro group, or a halogen atom. The ring formed together by the adjacent groups of R³ to R⁶ includes similar rings to the aforementioned rings formed by R¹ and R². Among them, aromatic or non-aromatic 5- to 12-membered rings are specifically preferred.

The imide compounds represented by Formula (1) can be prepared by a conventional imidation process (a process for the formation of an imide), such as a process that comprises the steps of allowing a corresponding acid anhydride to react with hydroxylamine NH₂OH for ring-opening of an acid anhydride group, and closing the ring to form an imide.

Examples of acid anhydrides include succinic anhydride, maleic anhydride, and other saturated or unsaturated aliphatic dicarboxylic anhydrides; tetrahydrophthalic anhydride, hexahydrophthalic anhydride (1,2-cyclohexanedicarboxylic anhydride), 1,2,3,4-cyclohexanetetracarboxylic 1,2-dianhydride, and other saturated or unsaturated non-aromatic cyclic polycarboxylic anhydrides (alicyclic polycarboxylic anhydrides); HET anhydride (chlorendic anhydride), himic anhydride, and other bridged cyclic polycarboxylic anhydrides (alicyclic polycarboxylic anhydrides); phthalic anhydride, tetrabromophthalic anhydride, tetrachlorophthalic anhydride, nitrophthalic anhydride, trimellitic anhydride, methylcyclohexenetricarboxylic anhydride, pyromellitic anhydride, mellitic anhydride, 1,8;4,5-naphthalenetetracarboxylic dianhydride, and other aromatic polycarboxylic anhydrides.

Preferred imide compounds include, for example, imide compounds derived from aliphatic polycarboxylic anhydrides such as N-hydroxysuccinimide and N-hydroxymaleimide; and imide compounds derived from alicyclic polycarboxylic anhydrides or aromatic polycarboxylic anhydrides such as
N-hydroxyhexahydrophthalimide,
N,N'-dihydroxycyclohexanetetracarboximide,
N-hydroxyphthalimide, N-hydroxytetrabromophthalimide,
N-hydroxytetrachlorophthalimide, N-hydroxychlorendimide,
N-hydroxyhimimide, N-hydroxytrimellitimide,
N,N'-dihydroxypyromellitimide, and
N,N'-dihydroxynaphthalenetetracarboximide.

Typically preferred imide compounds include N-hydroxyimide compounds derived from alicyclic polycarboxylic anhydrides or aromatic polycarboxylic anhydrides, of which N-hydroxyphthalimide and other N-hydroxyimide compounds derived from aromatic polycarboxylic anhydrides are specifically preferred. The imide compounds of Formula (1) can be used alone or in combination.

The imide compounds can be used as being supported on a carrier. As such carriers, activated carbon, zeolite, silica, silica-alumina, bentonite, and other porous carriers are frequently employed.

The amount of the imide compound can be selected depending on the types of the substrate and reaction and is generally from 0.0001 to 1 mole, preferably from 0.001 to 0.5 mole, more preferably from 0.01 to 0.4 mole, and often from 0.05 to 0.35 mole, relative to 1 mole of the substrate.

### [Promoter (Co-catalyst)]

In the inventive process, a promoter (co-catalyst) can be used in combination with the imide compound. The combination use of the imide compound with the promoter can improve or enhance the rate and/or selectivity of the reaction in some cases. Each of promoters can be used alone or in combination.

The promoters for use in the invention include compounds each containing a transition metal or a Group 13 element of the Periodic Table of Elements, and examples of such compounds include oxides, hydrides, nitrides, oxoacids or salts thereof, oxoacid esters, heteropolyacids or salts thereof, organic acid salts, inorganic acid salts, halides, and complexes. Elements of the transition metals include, for example, Group 3 elements (e.g., scandium Sc, yttrium Y; cerium Ce, samarium Sm, and other lanthanoid elements; and actinium Ac and other actinoid elements), Group 4 elements (e.g., titanium Ti and zirconium Zr), Group 5 elements (e.g., vanadium V and niobium Nb), Group 6 elements (e.g., chromium Cr, molybdenum Mo, and tungsten W), Group 7 elements (e.g., manganese Mn, technetium Tc, and rhenium Re), Group 8 elements (e.g., iron Fe and ruthenium Ru), Group 9 elements (e.g., cobalt Co and rhodium Rh), Group 10 elements (e.g., nickel Ni, palladium Pd, and platinum Pt), and Group 11 elements (e.g., copper Cu), of the Periodic Table of Elements. Preferred elements include Ce, V, Nb, Cr, Mo, W, Mn, Fe, Ru, Co, Rh, Ni, and Cu. Group 13 elements of the Periodic Table of Elements include, for example, boron B and aluminium Al.

The reaction system may further comprise, as promoters, azobisisobutyronitrile (AIBN) and other polymerization initiators, radical generators, or radical reaction accelerators [e.g., nitric acid, nitrous acid or salts of these acids, halogens (e.g., chlorine, and bromine), peracids, peroxides, and aldehydes (e.g., in the production of a carboxylic acid, an aldehyde corresponding to the carboxylic acid) . The presence of such components in the reaction system may accelerate or enhance the reaction in some cases. The reaction may be inhibited at a high concentration of the substrate, but the addition of AIBN or other polymerization initiators or the aldehydes or other compounds mentioned above permits the reaction to smoothly proceed even at a high concentration of the substrate.

The amount of the promoter depends on the types of the promoter and reaction, and is generally from 0.00001 to 0.8 mole, preferably from 0.0001 to 0.3 mole, and more preferably from 0.001 to 0.1 mole, relative to 1 mole of the substrate.

### [Substrates and Reaction Products]

Compounds for use as the reaction materials (substrates) in the present invention include a variety of compounds each having a site or moiety that can undergo oxidation with molecular oxygen, carboxylation, nitration, sulfonation, or carbon-carbon bond formation reactions (e.g., acylation, and radical coupling) in the presence of the imide compound catalyst. The compounds as described in, for example, the references indicated in Description of the Related Art can be referred to and incorporated in the present invention. Each of these compounds can be used alone or in combination.

Illustrative substrates are hydrocarbons, alcohols, aldehydes, ketones, amines, heterocyclic compounds, thiols, sulfides, and amides. Among them, hydrocarbons, alcohols, aldehydes and ketones are preferred as the substrates.

Examples of hydrocarbons include saturated or unsaturated aliphatic hydrocarbons which may have a substituent, saturated or unsaturated alicyclic hydrocarbons which may have a substituent, completely or partially hydrogenated condensed polycyclic hydrocarbons and other condensed cyclic hydrocarbons each containing a non-aromatic ring, bridged cyclic hydrocarbons each containing a tertiary carbon atom (a methine carbon), and aromatic hydrocarbons each having a methyl group or methylene group bonded to an aromatic ring.

Examples of saturated or unsaturated aliphatic hydrocarbons include butane, isobutane, pentane, hexane, octane, decane, and other C₄-C₂₀ saturated hydrocarbons; 2-butene, isobutene, and other C₄-C₂₀ olefin hydrocarbons; butadiene (1,3-butadiene), isoprene (2-methyl-1,3-butadiene), and other conjugated dienes, and other linear or branched aliphatic hydrocarbons, of which isobutane and other branched saturated hydrocarbons, isobutene and other branched unsaturated hydrocarbons, butadiene, isoprene, and other conjugated dienes are preferred.

Examples of saturated or unsaturated alicyclic hydrocarbons include cyclobutane, cyclopentane, cyclohexane, cycloheptane, cyclooctane, cyclononane, cyclodecane, cycloundecane, cyclododecane, cyclotridecane, cyclotetradecane, cyclooctadecane, cycloicosane, cyclodocosane, cyclotetracosane, cyclotriacontane, and other cycloalkanes; cyclopropene, cyclobutene, cyclopentene, cyclohexene, cycloheptene, cyclooctene, cyclononene, cyclodecene, and other cyclic olefins; cyclopentadiene, 1,3-cyclohexadiene, 1,4-cyclohexadiene, 1, 3-cycloheptadiene and other cycloheptadienes, 1,5-cyclooctadiene and other cyclooctadienes, and other cycloalkadienes; cyclooctatrienes, and other cycloalkatrienes; cyclooctatetraenes, and other cycloalkatetraenes; limonene, α-terpinen, β-terpinen, γ-terpinen, terpinolene, 1-p-menthene, 3-p-menthene, cis-carveol, trans-carveol, α-cedrene, valencene, isolongifolene, and other terpenes. Preferred alicyclic hydrocarbons include alicyclic hydrocarbons each having a ring with from 3 to 30 members, preferably from 3 to 25 members, and typically from 3 to 20 members (e.g., from 5 to 20 members, and specifically from 5 to 16 members). Of the terpenes, polycyclic compounds are also categorized as polycyclic hydrocarbons.

The condensed polycyclic hydrocarbons, bridged cyclic hydrocarbons, and other polycyclic hydrocarbons include compounds having at least one methylidine group (i.e., a methine carbon-hydrogen bond; -CH<) at the bridgehead position and/or at the junction position (a junction position between a ring and another). Examples of completely or partially hydrogenated condensed polycyclic hydrocarbons, and other condensed hydrocarbons each having a non-aromatic ring include acenaphthene, fluorene, tetralin, indene, indan, perhydroanthracene, perhydrophenanthrene, perhydrophenalene, perhydroacenaphthylene, decalin, and hexahydroindan. To these compounds, a 5- to 8-membered ring (specifically a 5-or 6-membered ring) is condensed in many cases.

Examples of bridged cyclic hydrocarbons include pinane, pinene, bornane, norbornane, norbornene, bicyclo[3.2.1]octane, bicyclo[4.3.2]undecane, and other bicyclic hydrocarbons; adamantane, exotricyclo[5.2.1.0^{2,6}] decane, endotricyclo[5.2.1.0^{2,6}]decane, and other tricyclic hydrocarbons; tetracyclo[4.4.0.1^{2,5}.1^{7,10}]dodecane, and other tetracyclic hydrocarbons; as well as dicyclohexadiene, dicyclopentadiene, and other dimers of dienes, hydrogenated products of these dimers such as dicyclohexane and dicyclopentane, and derivatives of these compounds; and monocyclic monoterpenes, bicyclic monoterpenes, monocyclic sesquiterpenes, bicyclic sesquiterpenes, tricyclic sesquiterpenes, diterpenes, triterpenes, tetraterpenes, polyterpenes, and derivatives of these compounds, and other terpenes. As the bridged cyclic hydrocarbons, bicyclic, tricyclic or tetracyclic hydrocarbons each having from 7 to 16 carbon atoms constituting rings and preferably from 6 to 14 carbon atoms constituting rings are often used, such as pinane, bornane, norbornane, norbornene, and adamantane.

The aromatic hydrocarbons each having a methyl group or methylene group bonded to an aromatic ring may be any compounds each having at least one methyl group or methylene group substituted on an aromatic ring, and the aromatic ring may be any of aromatic hydrocarbon rings and aromatic heterocyclic rings. Examples of such compounds include toluene, o-, m- and p-xylenes, 1,2,3-trimethylbenzene, mesitylene, 1,2,3,4-tetramethylbenzene, durene, 4-t-butyl-1-methylbenzene, ethylbenzene, propylbenzene, cumene, o-, m- and p-ethyltoluenes, 1-methylnaphthalene, 2-methylnaphthalene, 1,5-dimethylnaphthalene, 2,5-dimethylnaphthalene, 1-methylanthracene, 2-methylanthracene, 9-methylanthracene, 4,4'-dimethylbiphenyl, dibenzyl, diphenylmethane, and triphenylmethane. Among them, C₆-C₁₀ aromatic hydrocarbons each having from one to four substituted methyl groups per molecule are typically preferred.

The hydrocarbons may have at least one substituent depending on the types of the hydrocarbons. Examples of substituents include halogen atoms, alkyl groups, alkenyl groups, aryl groups, heterocyclic groups, oxo group, hydroxyl group, alkoxy groups, hydroxyalkyl groups, carboxyl group, alkoxycarbonyl group, acyl groups, amino group, substituted amino groups, cyano group, and nitro group.

Preferred hydrocarbons include (1) conjugated dienes such as butadiene and isoprene, (2) compounds each having a carbon-hydrogen bond at the adjacent position to an unsaturated bond, such as 2-butene and other C₄-C₂₀ olefinic hydrocarbons, (3) alicyclic hydrocarbons such as cyclohexane, methylcyclohexane, and other cycloalkanes each having a 5- to 16-membered ring; cyclohexene and other cycloalkenes each having a 5- to 16-membered ring; and valencene and other terpenes, (4) condensed cyclic compounds each having a non-aromatic ring (e.g., a cycloalkane ring or heterocyclic ring), such as decalin, tetralin and fluorene, (5) bridged cyclic hydrocarbons containing a tertiary carbon atom (a methine carbon), such as adamantane and norbornene, (6) aromatic hydrocarbons each having a methyl group or methylene group bonded to an aromatic ring, such as toluene, o-, m- and p-xylenes, p-t-butyltoluene, and other C₆-C₁₀ aromatic hydrocarbons each having from one to four methyl groups, and diphenyl methane and other aromatic hydrocarbons each having a methylene group bonded to an aromatic ring.

Oxidation of the hydrocarbons by oxygen in the presence of the imide compound catalyst yields corresponding oxides such as alcohols, aldehydes, ketones, carboxylic acids, epoxy compounds, lactones, acid anhydrides, acetals, or esters. For example, oxidation of conjugated dienes yields corresponding alkenediols. For example, oxidation of butadiene yields butenediols such as cis-isomer or trans-isomer of 2-butene-1,4-diol or 1-butene-3,4-diol, in which the positions of hydroxyl groups are not specifically limited. Oxidation of a compound having a carbon-hydrogen bond at the adjacent position to an unsaturated bond permits the adjacent position to the unsaturated bond to be oxidized. Oxidation of an alicyclic hydrocarbon introduces a hydroxyl group or oxo group into the ring, and under some conditions, the ring is further oxidatively cleaved to thereby yield, for example, a dicarboxylic acid. Oxidation of a condensed cyclic compound containing a non-aromatic ring introduces a hydroxyl group or oxo group into the non-aromatic ring, and under some conditions, the ring is further cleaved to thereby yield, for example, a dicarboxylic acid. Oxidation of a bridged cyclic hydrocarbon containing a tertiary carbon atom (a methine carbon) introduces a hydroxyl group into the tertiary carbon atom (e.g., the bridgehead position), or, under some reaction conditions, introduces an oxo group into the adjacent position to the tertiary carbon atom. Oxidation of an aromatic hydrocarbon having a methyl group or methylene group bonded to an aromatic ring permits the methyl group or methylene group to be oxidized to thereby yield a corresponding alcohol, aldehyde, ketone or carboxylic acid depending on conditions.

The reactions of the hydrocarbons with oxygen and carbon monoxide, nitrogen oxides (e.g., NO, NO₂, N₂O₃), sulfur oxides (e.g., SO₂), 1,2-dicarbonyl compounds, or compounds that can undergo a radical carbon-carbon bond formation reaction in the presence of the imide compound catalyst yield corresponding carboxylic acids, nitro compounds, organic sulfur acids (e.g., sulfonic acid), acylation reaction products (aldehydes, ketones), or products of carbon-carbon bond formation reaction or derivatives thereof (e.g., oxides, or lactones and other cyclized products), respectively. For example, the reactions of bridged cyclic hydrocarbons containing a tertiary carbon atom (a methine carbon) with oxygen and carbon monoxide, nitrogen oxides (e.g., NO, NO₂, N₂O₃), sulfur oxides (e.g., SO₂), 1,2-dicarbonyl compounds, or compounds that can undergo radical carbon-carbon bond formation reaction in the presence of the imide compound catalyst yield compounds in which a carboxyl group, nitro group, sulfo group, acyl group, hydrocarbon group or the like is introduced into the tertiary carbon atom, or derivatives thereof.

The compounds that can undergo a radical carbon-carbon bond formation reaction include, for example, unsaturated compounds, compounds each having a methine carbon atom, and other compounds that can trap radicals. Examples of such unsaturated compounds include (meth)acrylic esters, crotonic esters, cinnamic esters, maleic esters, fumaric esters, styrene and other unsaturated compounds each having an electron attracting group at the adjacent position to a carbon-carbon unsaturated bond (e.g., active olefins); methylacetylene and other compounds each having a carbon-carbon triple bond; compounds each having an aromatic carbon ring such as benzene ring; ketenes; isocyanate or thiocyanate compounds; propylene, 1-octene, and other inactive olefins. Examples of compounds each having a methine carbon atom include decalin, adamantane, and other bridged cyclic compounds; 1-methylcyclohexane and other non-aromatic cyclic compounds each having a hydrocarbon group bonded to a ring; and isobutane and other chain hydrocarbons each having a tertiary carbon atom. These compounds form a carbon-carbon bond with the substrates at the position of a carbon atom constituting the unsaturated bond or of the methine carbon atom.

The alcohols for use as the substrates include alcohol derivatives of the hydrocarbons, such as aliphatic monohydric alcohols, aliphatic polyhydric alcohols, alicyclic monohydric alcohols, alicyclic polyhydric alcohols, and aromatic alcohols.

Examples of aliphatic monohydric alcohols include methanol, ethanol, 1-propanol, isopropanol, 1-butanol, isobutanol, 1-pentanol, 2-pentanol, neopentyl alcohol, 1-hexanol, 1-octanol, 1-decanol, 1-dodecanol, myristyl alcohol, 1-hexadecanol, and other C₁-C₂₀ saturated aliphatic alcohols; allyl alcohol, crotyl alcohol, propargyl alcohol, citronellol, geraniol, and other unsaturated aliphatic alcohols. Examples of aliphatic polyhydric alcohols include ethylene glycol, propylene glycol, trimethylene glycol, 1,2-butanediol, 1,3-butanediol, 1,4-butanediol, 1,6-hexanediol, 2,5-hexanediol, neopentyl glycol, pinacol, and glycerin. Examples of alicyclic monohydric alcohols include cyclopentanol, cyclohexanol, cycloheptanol, cyclooctanol, cyclodecanol, cycloundecanol, cyclododecanol, cyclotetradecanol, cycloicosanol, methylcyclohexanol, cyclohexen-1-ol, cyclocten-1-ol, cyclogeraniol, borneol, menthol, and other alicyclic monohydric alcohols each having a 5- to 30-membered ring. Preferred alicyclic monohydric alcohols are compounds each having a 5- to 30-membered ring, more preferably a 5- to 25-membered ring, and typically a 5- to 20-membered ring (e.g., a 5- to 16-membered ring). Examples of alicyclic polyhydric alcohols include 1,2-cyclohexanediol and 1,4-cyclohexanediol, and examples of aromatic alcohols include benzyl alcohol, salicyl alcohol, benzhydrol, and phenethyl alcohol.

Of these alcohols, primary or secondary alcohols are preferred, which may be any of aliphatic alcohols, alicyclic alcohols, and aromatic alcohols.

Preferred alcohols include (1) compounds each having a hydroxyl group at the adjacent position to an unsaturated bond (e.g., allyl alcohol, benzyl alcohol, benzhydrol, and other unsaturated aliphatic alcohols and aromatic alcohols), (2) alicyclic alcohols (e.g., cyclohexanol, methylcyclohexanol, and other C₅-C₁₆ cycloalkanols), and (3) alicyclic alcohols each having a tertiary carbon atom (a methine carbon) (e.g., borneol).

Oxidation of these alcohols by oxygen in the presence of the imide compound catalyst yields corresponding aldehydes, ketones, or carboxylic acids. For example, oxidation of alicyclic alcohols yields corresponding alicyclic ketones or polycarboxylic acids depending on the degree of oxidation. For example, oxidation of 2-methylcyclohexanol yields 2-methylcyclohexanone, or further, 2-methyladipic acid. The reactions of primary or secondary alcohols with the compounds that can undergo a radical carbon-carbon bond formation reaction in the presence of the imide compound catalyst yield corresponding products of carbon-carbon bond formation reaction or derivatives thereof (e.g., oxides, lactones and other cyclized products). In this case, a carbon-carbon bond is formed at the position of a carbon atom carrying a hydroxyl group of the primary or secondary alcohols.

The aldehydes for use as the substrates include aldehyde derivatives of the hydrocarbons, such as formaldehyde, acetaldehyde, propionaldehyde, butyraldehyde, hexanal, octanal, nonal, and other C₁-C₂₀ saturated aliphatic aldehydes; acrolein, geranial (α-citral), citronellal, and other unsaturated aliphatic aldehydes; glyoxal, malonaldehyde, succinaldehyde, glutaraldehyde, adipaldehyde, pimelaldehyde, suberaldehyde, sebacaldehyde, and other aliphatic polyaldehydes, and other aliphatic aldehydes; as well as benzaldehyde, oxybenzaldehyde, cinnamaldehyde, salicylaldehyde, anisaldehyde, 1-naphthylaldehyde, vanillin (vanyllaldehyde), phthalaldehyde, isophthalaldehyde, terephthalaldehyde, and other aromatic aldehydes; formylcyclohexane, and other alicyclic aldehydes; and nicotinaldehyde, furfural, and other heterocyclic aldehydes.

Oxidation of the aldehydes by oxygen in the presence of the imide compound catalyst yields corresponding carboxylic acids. For example, oxidation of adipaldehyde yields adipic acid.

The ketones for use as the substrates include ketone derivatives of the hydrocarbons, such as aliphatic ketones, alicyclic ketones, aromatic ketones, and heterocyclic ketones. Examples of aliphatic ketones include acetone, methyl ethyl ketone, diethyl ketone, dipropyl ketone, methyl propyl ketone, methyl butyl ketone, pinacolone, and other C₂-C₂₀ aliphatic ketones. Examples of alicyclic ketones include cyclobutanone, cyclopentanone, cyclohexanone, cyclooctanone, cyclononanone, cyclodecanone, cycloundecanone, cyclododecanone, cyclotetradecanone, cyclooctadecanone, cycloicosanone, 2-methylcyclohexanone, 2-ethylcyclohexanone, 2,6-dimethylcyclohexanone, 4-chlorocyclohexanone, 4-methoxycyclohexanone, cyclohexanediones, cyclopentenone, cyclohexenones, cyclooctenones, cyclodecenones, menthone, camphor, and other alicyclic ketones (cyclic ketones) each having a 4- to 30-membered ring. Preferred alicyclic ketones include compounds each having a 5- to 20-membered ring, of which compounds each having a 5- to 16-membered ring are typically preferred. Examples of aromatic ketones include acetophenone, propiophenone, benzophenone, deoxybenzoin, and 1-naphthalenone. Examples of heterocyclic ketones include inden-1-one, 1,2,3-indantrione, fluolen-9-one, 4-pyranone, and other heterocyclic ketones.

Oxidation of the ketones by oxygen in the presence of the imide compound catalyst yields corresponding carboxylic acids. For example, oxidation of diethyl ketone yields acetic acid and propionic acid, and oxidation of cyclooctanone yields suberic acid.

The amines for use as the substrates are preferably primary or secondary amines, such as methylamine, ethylamine, propylamine, butylamine, dimethylamine, diethylamine, dibutylamine, ethylenediamine, 1,4-butanediamine, hydroxylamine, ethanolamine, and other aliphatic amines; cyclopentylamine, cyclohexylamine, and other alicyclic amines; benzylamine, toluidine, and other aromatic amines. Oxidation of the amines in the presence of the imide compound catalyst yields, for example, corresponding Shiff bases or oximes.

The heterocyclic compounds for use as the substrates include (a) non-aromatic heterocyclic compounds or condensed cyclic hydrocarbons each containing a non-aromatic heterocyclic ring (e.g., pyrane, pyrazoline, piperidine, piperazine, indoline, isoindoline, chromene, xanthene, chroman, and isochroman), and the aforementioned non-aromatic heterocyclic compounds or condensed cyclic hydrocarbons each containing a non-aromatic heterocyclic ring, which have an alkyl group (e.g., methyl, ethyl, and other alkyl groups each having from 1 to 6 carbon atoms) substituted on the non-aromatic heterocyclic ring; (b) heterocyclic compounds each having an aromatic heterocyclic ring and having a methyl group or methylene group at the adjacent position to the aromatic heterocyclic ring (e.g., 2-methylfuran, 2,5-dimethylfuran, 2-methylthiophene, 2,5-dimethylthiophene, 2-methylpyridine, 3-methylpyridine, 4-methylpyridine, 2,5-dimethylpyridine, 3-ethylpyridine, 2-methylquinoline, and other heterocyclic compounds each having an alkyl group containing from 1 to 6 carbon atoms substituted on an aromatic heterocyclic ring, which aromatic heterocyclic ring has one to three hetero atoms selected from among oxygen atoms, sulfur atoms and nitrogen atoms.

Oxidation of these heterocyclic compounds yields corresponding alcohols, ketones, or carboxylic acids. For example, oxidation of the heterocyclic compounds (a) permits a methylene group at the adjacent position to a hetero atom (e.g., oxygen, sulfur or nitrogen atom) in the non-aromatic heterocyclic ring to convert into a carbonyl group to thereby yield corresponding compounds each having a carbonyl group. Upon oxidation of the heterocyclic compounds (b), the compounds having a methyl group at the adjacent position to the aromatic heterocyclic ring yield corresponding heterocyclic aldehydes or heterocyclic carboxylic acids, and the compounds having a methylene group at the adjacent position to the aromatic heterocyclic ring yield corresponding heterocyclic ketones.

Examples of thiols for use as the substrates include ethanethiol, and phenylmethanethiol. Examples of sulfides include dimethyl sulfide, methyl propyl sulfide, and diphenyl sulfide. Examples of amides include formamide and acetamide.

The combination use of two or more substrates can markedly improve the reaction rate of reactions such as oxidation reaction in some cases.

### [Reactions]

Reactions are performed in the presence or in the absence of solvents. Such solvents can be appropriately selected depending on the types of the substrate and reaction, and include, for example, acetic acid, propionic acid, and other organic acids; acetonitrile, propionitrile, benzonitrile, and other nitriles; formamide, acetamide, dimethylformamide (DMF), dimethylacetamide, and other amides; hexane, octane, and other aliphatic hydrocarbons; chloroform, dichloromethane, dichloroethane, carbon tetrachloride, chlorobenzene, trifluoromethylbenzene, and other halogenated hydrocarbons; nitrobenzene, nitromethane, nitroethane, and other nitro compounds; ethyl acetate, butyl acetate, and other esters; and mixtures of these solvents. Frequently used solvents are acetic acid and other organic acids, acetonitrile, benzonitrile, and other nitriles, trifluoromethylbenzene and other halogenated hydrocarbons, and ethyl acetate and other esters.

An important feature of the present invention is that the imide compound catalyst is added in installments to a reaction system to perform reaction. In this connection, the term "addition in installments" as used herein means that the catalyst is not added at once in a batch but is continuously (successively) or intermittently added to the reaction system in installments. The addition of the imide compound catalyst in installments to the reaction system improves the conversion of material compounds and/or suppresses side reactions to thereby improve the selectivity of target compounds, as compared with the addition of the imide compound catalyst at once to the reaction system. The catalytic activity may be deteriorated at a high concentration of the substrate, but the addition of the imide compound catalyst in installments can smoothly proceed the reaction to hereby yield the target compound in a high yield even at a high concentration of the substrate. The reasons why the addition of the catalyst in installments are not completely clarified, but are speculated as follows. When the catalyst is added at once in a batch to the reaction system, the catalyst deteriorates with a proceeding reaction, and the concentration of an effective catalytically active species falls short of an appropriate level. In contrast, when the catalyst is added in installments, a fresh catalyst is appropriately added to the reaction system, and the concentration of an effective catalytically active species can be maintained at an appropriate level till the completion of the reaction.

The imide compound catalyst may be added to the reaction system as intact or may be dissolved or dispersed in an appropriate solvent before addition. The imide compound catalyst may be added to the reaction system continuously or intermittently. Additionally, the promoter may also be added to the reaction system in installments.

A reaction temperature can be appropriately selected depending on the types of the substrate and of reaction, and is generally from 0°C to 300°C, preferably from 10°C to 250°C, and more preferably from 20°C to 200°C. The reaction can be performed at normal atmospheric pressure or under a pressure (under a load) . When the reaction is performed under a pressure (under a load), the pressure is generally from 1 to 100 atm (i.e., from 0.101 to 10.1 MPa) and preferably from 1.5 to 80 atm (i.e., from 0.152 to 8.08 MPa).

The reaction can be performed in a conventional system such as a batch system, semi-batch system or continuous system. After the completion of reaction, the reaction products can be easily separated and purified by a conventional technique such as filtration, concentration, distillation, extraction, crystallization, recrystallization, column chromatography, and other separation and purification means, or combinations of these means.

### Advantages

The invented process supplies an imide compound catalyst such as N-hydroxyphthalimide to a reaction system by a specific technique in the production of an organic compound with the use of the imide compound catalyst, and can therefore yield a target compound with a higher conversion or selectivity.

### EXAMPLES

The present invention will be illustrated in further detail with reference to several examples and comparative examples below.

### EXAMPLE 1

In a flask, 10 mmol of valencene, 1 mmol of N-hydroxyphthalimide, 0.2 mmol of cobalt(II) acetate tetrahydrate, 0.6 mmol of acetylacetonatocobalt(III), and 20 ml of acetonitrile were placed and were stirred at 40°C in an oxygen atmosphere (1 atm = 0.101 MPa) for 2 hours. Subsequently, further 1 mmol of N-hydroxyphthalimide was added to the mixture and the resulting mixture was stirred at 40°C for further 2 hours. The reaction mixture was subjected to gas chromatographic analysis to find that a target compound nootkatone in a yield of 62%, nootkatol in a yield of 1%, and valencene epoxide in a yield of 3% were formed with a conversion from valencene of 99%.

### COMPARATIVE EXAMPLE 1

The procedure of Example 1 was repeated, except that N-hydroxyphthalimide (2 mmol) was added at once at the beginning of reaction (total reaction time: 4 hours). The reaction mixture was subjected to gas chromatographic analysis to find that the target compound nootkatone in a yield of 52%, nootkatol in a yield of 10%, and valencene epoxide in a yield of 3% were formed with a conversion from valencene of 100%.

### EXAMPLE 2

In a flask, 3 g (22 mmol) of adamantane, 0.36 g (10% by mole) of N-hydroxyphthalimide, 0.010 g (0.4% by mole) of a 60% by weight nitric acid, 0.023 g (0.3% by mole) of acetylacetonatovanadium V(acac)₃, and 27 g of acetic acid were placed and were stirred at 55°C in an oxygen atmosphere (1 atm = 0.101 MPa) for 3 hours. Subsequently, the resulting mixture was cooled in a nitrogen atmosphere, and further 0.36 g (10% by mole) of N-hydroxyphthalimide was added to the mixture, and the reaction was continued under the same condition as above for further 3 hours. The reaction mixture was subjected to gas chromatographic analysis to find that 1-adamantanol with a selectivity of 50%, 1,3-adamantanediol with a selectivity of 31%, 1,3,5-adamantanetriol with a selectivity of 2%, and 2-adamantanone with a selectivity of 7% were formed. The total of selectivity of the adamantanols was 83%, and the conversion from adamantane was 80%.

### COMPARATIVE EXAMPLE 2

The procedure of Example 2 was repeated, except that N-hydroxyphthalimide (0.72 g; 20% by mole) was added at once at the beginning of the reaction (total reaction time: 6 hours). The reaction mixture was subjected to gas chromatographic analysis to find that 1-adamantanol with a selectivity of 45%, 1,3-adamantanediol with a selectivity of 27%, 1,3,5-adamantanetriol with a selectivity of 2%, and 2-adamantanone with a selectivity of 7% were formed. The total of selectivity of the adamantanols was 74%, and the conversion from adamantane was 79%.

### EXAMPLE 3

In a 1-L autoclave made of titanium equipped with a cooling tube, pressure regulator, and mass flow, 40.0 g (430 mmol) of β-picoline, 7.01 g (43 mmol) of N-hydroxyphthalimide, 1.05 g (4 mmol) of manganese(II) acetate [Mn(OAc)₂.4H₂O], 1.07 g (4 mmol) of cobalt(II) acetate [Co(OAc)₂.4H₂O], 8.00 g (75 mmol) of pyridine-3-carbaldehyde (nicotinaldehyde), and 342.9 g of acetic acid were placed and were stirred at 140°C at a pressure of 10 kgf/cm² (= 0.981 MPa) for 2 hours under flow of air (80 L(normal state)/hr). The resulting mixture was cooled in a nitrogen atmosphere, and further 7.01 g (43 mmol) of N-hydroxyphthalimide and 8.00 g (75 mmol) of pyridine-3-carbaldehyde (nicotinaldehyde) were added thereto, and the reaction was continued under the same condition as above for further 2 hours. The reaction mixture was analyzed by gas chromatography and high performance liquid chromatography to find that nicotinic acid was formed in a yield of 67.7% with a selectivity of 95.7%, and that the conversion from β-picoline was 70.7%.

### COMPARATIVE EXAMPLE 3

The procedure of Example 3 was repeated, except that N-hydroxyphthalimide (14.00 g; 86 mmol) was added at once in a batch at the beginning of the reaction (total reaction time: 4 hours). The reaction mixture was analyzed by gas chromatography and high performance liquid chromatography to find that nicotinic acid was formed in a yield of 58.9% with a selectivity of 95.3%, and that the conversion from β-picoline was 61.8%.

## Claims

1. A process for producing an organic compound by a reaction selected from the group consisting of oxidation reactions, carboxylation reactions, nitration reactions, sulfonation reactions, and carbon-carbon bond formation reactions, by catalysis of an imide compound represented by the following Formula (1): 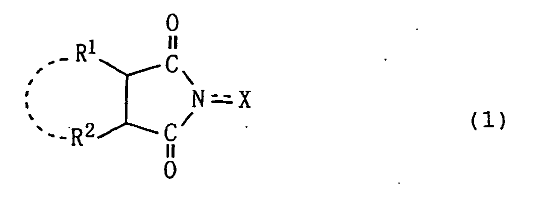 wherein each of R¹ and R² is independently a hydrogen atom, a halogen atom, a straight- or branched-chain alkyl group having from 1 to 10 carbon atoms, a phenyl or naphthyl group, a cyclopentyl, cyclohexyl, or cyclooctyl group, a hydroxyl group, an alkoxy group having from 1 to 10 carbon atoms, a carboxyl group, an alkoxycarbonyl group having from 1 to 10 carbon atoms in the alkoxy moiety, or an acyl group having from 1 to 6 carbon atoms, where R¹ and R² may be combined to form a double bond or an aromatic or non-aromatic ring having from 5 to 12 members; X is an oxygen atom or a hydroxyl group; and one or two of N-substituted cyclic imido group indicated in the formula may be further formed on said R¹, R², or on the double bond or aromatic or non-aromatic ring formed together by R¹ and R², **characterised in that** the imide compound catalyst is added to a reaction system in installments.

2. Use of an imide compound of Formula (1) as defined in claim 1 as a catalyst for the production of an organic compound, wherein said imide compound catalyst is added to a reaction system in installments to perform a reaction selected from the group consisting of oxidation reactions, carboxylation reactions, nitration reactions, sulfonation reactions, and carbon-carbon bond formation reactions.

## Patentansprüche

1. Verfahren zur Herstellung einer organischen Verbindung durch eine Reaktion, ausgewählt aus der Gruppe, bestehend aus einer Oxidation, einer Carboxylierung, einer Nitrierung, einer Sulfonierung und einer Reaktion, bei der eine Kohlenstoff-Kohlenstoff-Bindung gebildet wird, wobei die Reaktion mit einer Imidverbindung katalysiert wird, dargestellt durch die folgende Formel (1): 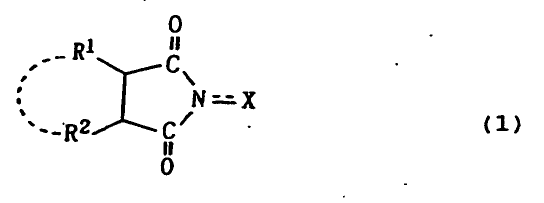 worin R¹ und R² jeweils unabhängig voneinander ein Wasserstoffatom, ein Halogenatom, eine geradkettige oder verzweigte Alkylgruppe mit 1 bis 10 Kohlenstoffatomen, eine Phenylgruppe oder eine Naphthylgruppe, eine Cyclopentylgruppe, eine Cyclohexylgruppe oder eine Cyclooctylgruppe, eine Hydroxygruppe, eine Alkoxygruppe mit 1 bis 10 Kohlenstoffatomen, eine Carboxygruppe, eine Alkoxycarbonylgruppe mit 1 bis 10 Kohlenstoffatomen im Alkoxyrest oder eine Acylgruppe mit 1 bis 6 Kohlenstoffatomen bedeuten, wobei R¹ und R² miteinander verbunden sein können, um eine Doppelbindung oder einen aromatischen oder nichtaromatischen Ring mit 5 bis 12 Gliedern zu bilden; X ist ein Sauerstoffatom oder eine Hydroxygruppe; und eine oder zwei N-substituierte cyclische Imidogruppen, die in der Formel dargestellt werden, können weiterhin an die Gruppen R¹ oder R² oder an die Doppelbindung oder an den aromatischen oder nichtaromatischen Ring, gebildet durch R¹ und R², gebunden sein, **dadurch gekennzeichnet, dass** die Imidverbindung, die als Katalysator verwendet wird, portionsweise zu dem Reaktionssystem gegeben wird.

2. Verwendung einer Imidverbindung der Formel (1) wie in Anspruch 1 definiert als Katalysator bei der Herstellung einer organischen Verbindung, wobei die Imidverbindung, die als Katalysator verwendet wird, portionsweise zu dem Reaktionssystem gegeben wird, wobei die organische Verbindung durch eine Reaktion hergestellt wird, ausgewählt aus der Gruppe, bestehend aus einer Oxidation, einer Carboxylierung, einer Nitrierung, einer Sulfonierung und einer Reaktion, bei der eine Kohlenstoff-Kohlenstoff-Bindung gebildet wird.

## Revendications

1. Procédé de production d'un composé organique par une réaction choisie dans le groupe constitué par les réactions d'oxydation, les réactions de carboxylation, les réactions de nitration, les réactions de sulfonation et les réactions de formation d'une liaison carbone-carbone, par catalyse d'un composé imide représenté par la formule (1) suivante : 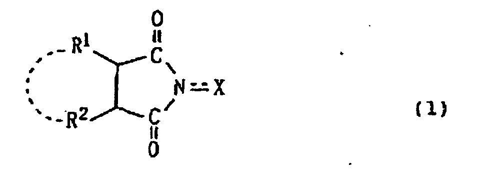 dans laquelle chacun de R¹ et R² est indépendamment un atome d'hydrogène, un atome d'halogène, un groupe alkyle à chaîne droite ou ramifiée comportant 1 à 10 atomes de carbone, un groupe phényle ou naphtyle, un groupe cyclopentyle, cyclohexyle ou cyclooctyle, un groupe hydroxyle, un groupe alcoxy comportant 1 à 10 atomes de carbone, un groupe carboxyle, un groupe alcoxycarbonyle comportant 1 à 10 atomes de carbone dans le groupement alcoxy, ou un groupe acyle comportant 1 à 6 atomes de carbone, où R¹ et R² peuvent être combinés pour former une double liaison ou un cycle aromatique ou non aromatique comprenant 5 à 12 membres ; X est un atome d'oxygène ou un groupe hydroxyle ; et un ou deux des groupes imido cycliques N-substitués présentés sur la formule peuvent être en outre formés sur lesdits radicaux R¹, R² ou sur la double liaison ou sur le cycle aromatique ou non aromatique formé avec R¹ et R², **caractérisé en ce que** le catalyseur de type composé imide est ajouté au système réactionnel en plusieurs fois.

2. Utilisation d'un composé imide de formule (1) tel que défini dans la revendication 1 comme catalyseur pour la production d'un composé organique, dans laquelle ledit catalyseur de type composé imide est ajouté à un système réactionnel en plusieurs fois pour réaliser une réaction choisie dans le groupe constitué par les réactions d'oxydation, les réactions de carboxylation, les réactions de nitration, les réactions de sulfonation et les réactions de formation d'une liaison carbone-carbone.
